## Europäisches Patentamt
## European Patent Office
## Office européen des brevets

(19)

(11) Numéro de publication: **0 157 672**
**B1**

(12) # FASCICULE DE BREVET EUROPÉEN

(45) Date de publication du fascicule du brevet:
**12.07.89**

(51) Int. Cl.⁴: **G 01 N 33/537,** G 01 N 33/92, G 01 N 33/68, G 01 N 33/577, G 01 N 33/543

(21) Numéro de dépôt: **85400419.9**

(22) Date de dépôt: **05.03.85**

(54) Procédé de dosage immunologique d'antigènes, d'anticorps ou d'haptènes, son utilisation pour le dosage des lipoprotéines et de l'alpha-foetoprotéine et trousses de dosage pour la mise en oeuvre de ce procédé.

(30) Priorité: **09.03.84 FR 8403675**

(43) Date de publication de la demande:
**09.10.85 Bulletin 85/41**

(45) Mention de la délivrance du brevet:
**12.07.89 Bulletin 89/28**

(84) Etats contractants désignés:
**BE CH DE GB IT LI NL SE**

(56) Documents cité:
**EP-A-0 062 892
WO-A-80/02460
WO-A-82/01773
US-A-4 098 876
US-A-4 298 687**

**JOURNAL OF IMMUNOLOGICAL METHODS, volume 42, no. 1, 1981, Elsevier, North Holland Biomedical Press, NL; M. UOTILA: "Two-site sandwich enzyme immunoassay with monoclonal antibodies to human alpha-fetoprotein", pages 11-15,
CLINICAL CHEMISTRY, volume 2, no. 1, janvier 1983, pages 115-119, Washington, US; G. DE GROOTE et al.: "Use of monoclonal antibodies to detect human placental alkaline phosphatase",**

(73) Titulaire: **Société anonyme: COMPAGNIE ORIS INDUSTRIE, 33, rue de la Fédération BP 510, F-75752 Paris Cédex (FR)**

(72) Inventeur: **Dedieu, Alain, 4, rue de l'Olivier, F-84000 Avignon (FR)**
Inventeur: **Jolu, Etienne, 2, Allée du Romarin, F-30200 Bagnols Sur Ceze (FR)**
Inventeur: **Lockhart, Campbell, 8, Allée du Romarin, F-30200 Bagnols Sur Ceze (FR)**

(74) Mandataire: **Pottier, Pierre Société BREVATOME, 25, Rue de Ponthieu, F-75008 Paris (FR)**

## EP 0 157 672 B1

### Description

La présente invention a pour objet un procédé de dosage immunologique d'antigènes, d'haptènes ou d'anticorps, utilisable en particulier pour le dosage des protéines plasmatiques telles que les lipoprotéines.

Depuis quelques années le dosage des lipoprotéines s'est developpé afin de mieux apprécier l'existence d'une dyslipidémie et en particulier les risques cardiovasculaires liés aux hyperlipidémies.

Dans ce but, on a tout d'abord dosé les lipides totaux (triglycérides et cholestérol) en circulation dans le sang, mais des études épidémiologiques ont très vite montré que la mesure de ces paramètres était imprécise. On s'est alors intéressé à certaines fractions de ces lipides portés par des apoprotéines particulières, et on a dosé par exemple le HDL cholestérol et le LDL cholestérol qui peuvent donner une indication plus précise sur l'existence d'une dyslipidémie.

Les lipoprotéines sont des complexes moléculaires composés de lipides et de protéines. Une de leurs particularités essentielles est d'avoir une densité plus faible que les autres protéines sériques, ce qui permet justement de les isoler des autres protéines et même de les classer par ultracentrifugation.

On peut donc classer ces lipoprotéines selon leur densité et isoler les fractions suivantes:

1° les chylomicroms de densité inférieure à 0,94 g/l qui sont les particules les plus grandes et les moins denses composées à environ 98 % de lipides;
2° les lipoprotéines de très faible densité, ayant une densité de 0,94 à 1,006;
3° les lipoprotéines de faible densité, ayant une densité de 1,006 à 1,063;
4° les lipoprotéines de haute densité ayant une densité de 1,063 à 1,21; et
5° les lipoprotéines de très haute densité.

Cependant, cette classification empirique basée sur les caractéristiques physicochimiques des lipoprotéines donne des classes très hétérogènes de par leur composition en protéines. Ainsi, le lipoprotéines de faible densité contiennent pour l'essentiel des apoprotéines B mais également des apoprotéines C et des apoprotéines E, et la fraction de lipoproténes de haute densité contient en majorité des apoprotéines $A_1$ et des apoprotéines $A_2$ mais également des apoprotéines C, des apoprotéines D et des apoprotéines E.

On s'est alors intéressé au rôle joué par ces apoprotéines dans le transport des lipides pour essayer de progresser dans la compréhension du métabolisme assez complexe de ces lipides. On s'est appliqué à démontrer que le dosage de certaines de ces protéines vectrices pouvait être un indicateur bien supérieur pour la détection des dyslipidémies.

Aussi, d'autres techniques de dosage se sont développées pour déterminer plus précisément la teneur du plasma sanguin en certaines apolipoprotéines.

Cependant, ces techniques de dosage posent certains problèmes en raison de la concentration élevée en apolipoprotéines du plasma sanguin et de la faible variation du taux de lipoprotéines entre les états dits normaux et les états dits pathologiques.

A titre d'exemple, on donne ci-après les concentrations plasmatiques des lipoprotéines et apolipoprotéines chez des sujets normolipidémiques de sexe masculin entre 25 et 40 ans.

| Lipoprotéines | | Apolipoprotéines | |
|---|---|---|---|
| mg/100 ml | | mg/100 ml | |
| VLDL | 80 | Apo-B | 80-100 |
| LDL | 276 | Apo-AI | 120-150 |
| $HDL_2$ | 86 | Apo-AII | 20-80 |
| $HDL_3$ | 254 | Apo-E | 10 |
| $HDL_2/HDL_3$ | 0,36 | Apo-CI | 5-10 |
| | | Apo-CII | 3-5 |
| | | Apo-CIII | 15-20 |
| | | Apo-D | 10-20 |
| | | Apo-F | 15 |

Aussi, pour obtenir une sensibilité satisfaisante, on a développé des procédés de dosage immunologique par exemple par immunodiffusion radiale, par immunoturbidimétrie et par immunonephélémétrie par rayons laser, ou encore par radioimmunologie ou par enzymoimmunologie comme cela est décrit par M. Rosseneu, R. Vercaemst, K.K. Steinberg et G.R. Cooper dans CLIN. CHEM. 29/3, 427 - 433 (1983).

Cependant, la plupart des techniques développées jusqu'à présent se heurtent à des inconvénients qui rendent leur usage mal aisé pour la routine d'un laboratoire de biologie clinique.

Ainsi, l'immunoélectrophorèse est une méthode difficilement automatisable. De plus, les prises d'échantillons recommandées (2 μl) ne sont pas compatibles avec des dosages en série.

La néphélémétrie par rayons laser n'oblige plus qu'à une dilution au 1/100è des échantillons à doser mais elle suppose l'acquisition d'un matériel spécifique et coûteux. De plus, des problèmes se posent lorsqu'on veut doser des échantillons particulièrement chargés en lipides.

2

Les dosages par radioimmunologie ou par enzymoimmunologie tels que ceux décrits dans US-A-4 098 876 et J. Immunol. Methods 1981, 42(1), p. 11 - 15, permettent d'obtenir une bonne sensibilité puisque ces dosages ont été mis au point pour détecter des quantités très faibles d'antigènes, mais ils sont mal adaptés au dosage des lipoprotéines qui se trouvent au contraire en quantité importante dans le sang (de l'ordre du gramme par litre).

Dans le brevet US-A-4 098 876, on détermine la présence et/ou la concentration d'une substance antigénique polyvalente dans un fluide par un procédé comprenant les étapes suivantes:

1) incuber l'échantillon de fluide contenant la substance à doser avec un anticorps marqué pour former un complexe immunochimique marqué,
2) incuber ce complexe avec des anticorps immobilisés pour former un second complexe marqué,
3) séparer la phase solide de l'échantillon,
4) déterminer le taux de marqueur dans le second complexe, et
5) comparer les résultats obtenus avec une courbe standard.

Ainsi, dans la première étape, on utilise une quantité d'antigène marqué suffisante pour complexer toute la substance à doser qui peut être présente dans l'échantillon.

L'article de J. Immunol. Methods, illustre un dosage immunologique de l'alpha-foetoprotéine humaine utilisant comme marqueur une enzyme et des anticorps monoclonaux. Dans ce cas, on incube les solutions d'antigènes avec des plaques en polystyrène revêtues d'anticorps monoclonaux, puis on les lave. On ajoute alors des anticorps marqués par la peroxydase de Raifort dans les récipients contenant les plaques qui ont été incubées avec l'antigène et, après deux heures d'incubation suivies de plusieurs lavages, on développe la couleur pour doser le taux d'enzyme marquée à la peroxydase de Raifort fixé sur les plaques. On peut aussi effectuer simultanément l'incubation de la solution d'antigène avec l'anticorps monoclonal fixé sur les plaques et l'anticorps marqué par la peroxydase de Raifort.

Aussi, le dosage des lipoprotéines par ces techniques oblige à des dilutions importantes de l'échantillon, qui sont incompatibles avec un travail en laboratoire d'analyses de routine. De plus, ces dilutions ne peuvent être que la source d'erreurs importantes.

On a toutefois développé un procédé de dosage enzymoimmunologique des apolipoprotéines B, qui permet de ne diluer les échantillons que 200 fois, mais la stabilité des réactifs utilisés dans cette methode n'étant pas assurée au delà de quelques jours, ceci la rend incompatible avec un travail de routine. Cette méthode est décrite dans Journal of Immunological Methods, 21 (1978) p. 317 - 324.

La présente invention a précisément pour objet un procédé de dosage immunologique qui permet d'obtenir une très bonne sensibilité sans exiger des dilutions élevées des échantillons à doser.

Le procédé selon l'invention, de dosage immunologique quantitatif d'une substance à l'aide d'au moins un réactif immunoactif comprend les étapes suivantes:

1) mettre en contact un échantillon contenant la substance à doser avec un réactif immunoactif marqué spécifique de ladite substance;
2) mettre en contact au moins une partie du milieu réactionnel ainsi obtenu avec une phase solide sur laquelle est fixé un réactif immunoactif spécifique de la substance à doser;
3) séparer le milieu réactionnel de la phase solide; et
4) déterminer la teneur en réactif immunoactif marqué de la phase solide, et il se caractérise en ce que l'on utilise dans la première étape une quantité de réactif immunoactif telle que la substance à doser se trouve en excès par rapport au réactif immunoactif marqué.

Selon une variante de mise en oeuvre du procédé de l'invention, on réalise simultanément les étapes 1) et 2) en mettant en contact la substance à doser simultanément avec le réactif immunoactif marqué et avec la phase solide sur laquelle est fixé le réactif immunoactif.

Selon l'invention, la substance à doser est un antigène, un haptène ou un anticorps, et le réactif immunoactif est un réactif possédant une activité immunologique, qui est capable de reconnaître la substance à doser et de se combiner avec elle.

Lorsque la substance à doser est un antigène ou un haptène, le réactif immunoactif est un anticorps spécifique de l'antigène ou de l'haptène.

Dans ce cas, le réactif immunoactif marqué est un anticorps marqué qui peut être un anticorps marqué soit par un radioélément, soit par un élément fluorescent, soit par une enzyme, par exemple un composé conjugué d'une enzyme et d'un anticorps spécifique de l'antigène ou de l'haptène à doser.

Le réactif immunoactif fixé sur la phase solide est également un anticorps qui peut être le même anticorps que celui constituant l'anticorps marqué. Toutefois, l'anticorps marqué et l'anticorps fixé sur la face solide peuvent provenir de sources différentes, à condition qu'ils soient tous deux capables de reconnaître le même antigène.

Selon un mode préféré de mise en oeuvre du procédé de l'invention pour le dosage d'antigènes ou d'haptènes, l'un au moins des anticorps utilisés est un anticorps monoclonal.

On décrit plus en détail, ci-après, la mise en oeuvre du procédé de l'invention pour le dosage immunologique d'un antigène. Dans ce cas, dans la première étape du procédé de l'invention, on réalise un marquage de l'antigène à doser par réaction de celui-ci avec l'anticorps marqué spécifique de cet antigène qui est présent en quantité constante mais telle que l'antigène soit en excès par rapport à l'anticorps. Aussi, à la fin de cette étape, on obtient un milieu réactionnel comprenant, d'une part, des molécules d'antigène libre en excès, et, d'autre part, des molécules d'antigène lié à l'anticorps marqué.

Dans la deuxième étape, lorsqu'on met en contact ce milieu réactionnel ou une partie de celui-ci avec une phase solide sur laquelle est fixé un anticorps spécifique des antigènes à doser, il y a compétition entre les molécules d'antigène lié à l'anticorps marqué et les molécules d'antigène libre pour les sites anticorps fixés sur la phase solide. A la fin de cette étape, les sites d'anticorps disponibles sur la phase solide seront pour l'essentiel occupés par les molécules d'antigène libre et par les molécules d'antigène lié à l'anticorps marqué et le rapport entre les molécules d'antigène libre fixées et les molécules d'antigène lié à l'anticorps marqué fixées sur cette phase solide dépendra de la quantité d'antigène libre présent dans le milieu réactionnel. Ainsi, plus la quantité d'antigene libre sera importante, plus la quantité d'antigène libre fixé sur la phase solide à la fin de la deuxième étape sera importante.

Comme on l'a vu précédemment, on peut aussi réaliser ces deux étapes simultanément en mettant en contact l'antigène avec l'anticorps marqué spécifique de cet antigène et avec la phase solide sur laquelle est fixé un anticorps spécifique de l'antigène à doser. Ainsi, on réalise au cours de la même étape le marquage de l'antigène à doser et la fixation de l'antigène sur la phase solide.

Dans la dernière étape du procédé, on détermine la teneur en anticorps marqué de la phase solide. Cette détermination dépend en particulier du marqueur utilisé. Lorsque le marqueur est un élément radioactif, on détermine la radioactivité de la phase solide ou éventuellement la radioactivité du milieu réactionnel séparé de la phase solide. Lorsque le marqueur est une enzyme, on révèle par une méthode colorimétrique la quantité d'enzyme fixée sur la phase solide et on détermine la densité optique du milieu dans un spectrophotomètre.

Après cette mesure, on connaît ainsi la quantité d'antigène lié à l'anticorps marqué, qui est fixé sur la phase solide et comme cette quantité dépend de la quantité d'antigène libre restant à la fin de la première étape, on peut en déduire la teneur en antigène de l'échantillon.

Pour obtenir cette valeur, on procède tout d'abord à un dosage d'échantillons ayant des concentrations connues en antigène, afin d'établir une courbe standard et on se reporte ensuite à la courbe standard pour déterminer la teneur en antigène de l'échantillon.

Le procédé de l'invention est particulièrement avantageux pour le dosage d'antigènes constitués par des protéines présentes dans un fluide biologique en concentration relativement importante.

En effet, le fait de réaliser, dans le procédé de l'invention, une compétition entre des protéines libres et des protéines liées à un anticorps marqué pour les sites d'un anticorps spécifique des protéines, nécessite d'opérer avec un excès de ces protéines dans la première étape. De ce fait, ce procédé est adapté au dosage d'échantillons ayant des concentrations relativement importantes en protéines à doser et il permet d'obtenir une sensibilité satisfaisante sans qu'il soit nécessaire de diluer de façon importante les échantillons à doser.

En revanche, lorsqu'on utilise les techniques de dosage immunologique dites par excès, la nécessité de travailler au cours de la première étape avec un excès d'anticorps, alors que cet anticorps fixé sur le support solide est en quantité limitée, oblige à des dilutions de l'antigène d'autant plus élevées que cet antigène est en quantité importante dans l'échantillon à doser.

Par ailleurs, dans le procédé de l'invention, on peut travailler avec des réactifs stables, et lorsqu'on utilise un anticorps marqué par une enzyme, il n'est pas nécessaire d'utiliser un appareillage particulier et onéreux puisqu il suffit d'un spectrophotomètre pour effectuer la mesure finale. De plus, cette méthode permet d'obtenir une sensibilité équivalente à la néphélémétrie et supérieure à la plupart des autres méthodes de dosage.

Le procédé de l'invention s'applique au dosage de différentes protéines, en particulier au dosage des protéines plasmatiques telles que les lipoprotéines, les immunoglobulines, les facteurs du complément et les glycoprotéines du sérum.

A titre d'exemple, les lipoprotéines peuvent être les apolipoprotéines B, $A_1$, $A_2$, C, D, E etc... et leurs combinaisons; les immunoglobulines peuvent être IgG, IgA, IgM, IgD, IgE, etc...; et les facteurs du complément peuvent être $C_1q$, $C_3c$, $C_4$, $C_5$ ou $C_3a$. Les glycoprotéines du sérum peuvent être l'orosomucoïde, l'haptoglobuline, l'alpha-2-macroglobuline, la coeruléoplasmine, la préalbumine, l'albumine, la transférine, la protéine liant le rétinol, l'alpha-l-antitrypsine, l'antithrombine-III, l'inhibiteur antitrypsine, l'hoemopescine, le fibrinogène et la bêta SP.

On peut aussi utiliser le procédé de l'invention pour doser le complexe Immun circulant, l'haptoglobine, la protéine C réactive, les hormones lactogènes placentaires, l'$\alpha$-foetoprotéine, la $C_1$ estérase, le $C_1$ estérase inhibiteur et l'$\alpha_1$-antichymotrypsine.

Le procédé de l'invention se révèle particulièrement intéressant pour réaliser le dosage quantitatif des lipoprotéines présentes dans le plasma sanguin. Dans ce cas, le procédé comprend les étapes suivantes:

1) mettre en contact un échantillon de plasma sanguin contenant les lipoprotéines à doser avec un composé conjugué d'un anticorps spécifique de ces lipoprotéines et d'une enzyme en quantité telle que les lipoprotéines à doser se trouvent en excès par rapport à l'anticorps;

2) mettre en contact au moins une partie du milieu réactionnel ainsi obtenu avec une phase solide sur laquelle est fixé le même anticorps spécifique des lipoprotéines à doser;

3) séparer le milieu réactionnel de la phase solide; et

4) déterminer la teneur en enzyme de la phase solide.

Selon une variante de mise en oeuvre de ce procédé, on réalise simultanément les deux étapes 1) et 2) en mettant en contact l'échantillon de plasma sanguin contenant les lipoprotéines à doser avec le composé conjugué de l'anticorps spécifique de ces lipoprotéines et d'une enzyme et avec la phase solide sur laquelle est fixé le même anticorps spécifique des lipoprotéines à doser.

L'enzyme utilisée est de préférence la peroxydase de Raifort.

Pour la réalisation du dosage, on commence tout d'abord, par effectuer le dosage d'échantillons dont on connaît la teneur en lipoprotéines afin d'établir la courbe étalon ou courbe standard qui donnera la densité optique (déterminée en fin d'opération) en fonction de la concentration en lipoprotéine.

En se reportant ensuite à cette courbe standard, on peut déterminer à partir de la valeur de la densité optique mesurée sur l'échantillon à doser la teneur en lipoprotéine cet échantillon.

Les différents réactifs utilisés pour mettre en oeuvre le procédé de l'invention sont préparés par des procédés classiques.

Ainsi, les échantillons standard ayant une concentration connue en lipoprotéines sont obtenus par dilution à partir de plasma humain normal, du point de vue du bilan lipidique, provenant d'un nombre limité de donneurs. On isole les lipoprotéines voulues par ultracentrifugation séquentielle en utilisant les méthodes décrites par Havel, R.J. and Coll. (1955), J.Clin. Bioch. 34,1, 345 - 353, Chapman and Coll. (1976) Atherosclerosis 25, 267 - 291.

Généralement le sang est collecté à partir de différents sujets à jeûn dans des tubes contenant de l'éthylène diamine tétraacétate de sodium à 1 mg/ml. On sépare ensuite le plasma par centrifugation pendant 20 min à 2500 g et on ajuste la densité du solvant à 1,050 avec du NaCl solide. On centrifuge alors dans une ultracentrifugeuse en effectuant plusieurs étapes de façon à obtenir les fractions de densités allant de 1,025 à 1,050. A partir de ces fractions, on isole les lipoprotéines voulues par des méthodes classiques, par exemple par ultracentrifugation dans le cas des lipoprotéines B, et on calibre la solution de lipoprotéines par une méthode analytique précise.

L'anticorps spécifique des lipoprotéines est préparé à partir des lipoprotéines isolées précédemment qui sont utilisées pour obtenir par immunisation chez un animal tel que le lapin, le mouton, etc... des immunoglobulines spécifiques antilipoprotéines humaines. Les immunoglobulines sont obtenues à partir du sérum de l'animal immunisé, purifiées par précipitation à l'acide caprylique et séparées par centrifugation. Le surnageant est récupéré par filtration et dyalisé une nuit à 4°C contre un tampon acétate. La solution est ensuite filtrée et sa pureté est testée par électrophorèse.

Le composé conjugué de l'enzyme et de l'anticorps spécifique des lipoprotéines à doser est fabriqué selon des techniques classiques, par exemple en utilisant la technique au périodate décrite par Wilson B.M. et Nakane P.K. dans "Recent developments in the periodate method of conjugating horseradish peroxidase (HRPO) to antibodies" - In Knapp W. Holubar K. and Wick G. (eds) - "Immunofluorescence and related staining techniques "New-York: Elsevie/North-Holland Biomedical Press, 1978, p. 215, lorsque l'enzyme est la peroxydase de Raifort, puis purifié par filtration sur gel.

Pour la mise en oeuvre du procédé de l'invention, la phase solide sur laquelle est fixé l'anticorps est généralement constituée par un dispositif à ailettes disposé à l'intérieur d'un tube en matière plastique. Ce dispositif à ailettes est généralement réalisé en polyamide, et on fixe l'anticorps sur celui-ci en introduisant dans le tube une solution des immunoglobulines purifiées antilipoprotéines humaines, obtenues par exemple chez le mouton, et en laissant cette solution au contact des ailettes pendant environ 4 heures. De préférence, on a activé avant les immunoglobulines selon la méthode décrite par Eiji Ishikawa, Yoshitaka Hamaguchi, Masayoshi Imagawa (III,8-preparation of antibody coated polystyrene balls for sandwich enzyme immunoassay-pp. 119 - 122 Enzyme Immunoassay Ishikawa, Kawai, Miyaki Igahu-Shoin, Tokyo (1981)).

Plusieurs tubes contenant la quantité voulue d'anticorps peuvent être préparés de cette façon.

Lorsque l'enzyme utilisée pour le marquage de l'anticorps est la peroxydase de Raifort, on utilise pour la révélation enzymatique un chromogène qui est reconstitué au moment de l'emploi à partir d'une poudre obtenue par lyophilisation d'une solution d'orthophénylène diamine dans du phosphate de sodium.

Pour la première étape du procédé de l'invention, on peut utiliser un tube en plastique ne comportant pas de dispositif à ailettes, ou le tube comportant un dispositif à ailettes décrit précédemment qui a été modifié de façon à présenter à sa partie supérieure une cupule en matière synthétique située au-dessus du dispositif à ailettes sur lequel est fixé l'anticorps. Cette cupule doit être facilement perforable afin de permettre l'écoulement du milieu réactionnel en fin de première étape sur la phase solide, c'est-à-dire sur le dispositif à ailetes présent à la partie inférieure du tube. Des tubes comportant un dispositif à ailettes utilisables dans le procédé de l'invention sont décrits en particulier dans le brevet français EN 78/050040 déposé au nom du Commissariat à l'Energie Atomique).

Les différents réactifs et matériels utilisés pour le dosage immunologique de l'invention peuvent être disposés dans une trousse qui comprendra:

- un système de tubes comportant chacun une phase solide, par exemple un dispositif à ailettes, revêtue dans les mêmes conditions d'immunoglobulines antilipoprotéines humaines. Ces tubes sont conservés à l'abri de l'humidité dans des emballages adéquats du type blister;

- une série de flacons contenant des échantillons standard qui couvriront la gamme de 0,1 à 0,005 g/l de lipoprotéines. Ces échantillons peuvent être conditionnés soit sous forme liquide avec adjonction de stabilisateurs adéquants tels que des antibiotiques, des antioxydants et un inhibiteur de protéase, soit sous forme lyophilisée, la lyophilisation étant effectuée en présence de sucrose;
- un flacon contenant le composé conjugué provenant du couplage des immunoglobulines antilipoprotéines humaines avec une enzyme telle que la peroxydase de Raifort. Ce composé peut être prédilué dans un tampon borate contenant du sérum de mouton délipoprotéiné et un conservateur tel que le thimérosal, sous forme liquide ou lyophilisée; et
- un flacon contenant un chromogène pour la révélation enzymatique. Le chromogène peut être présenté sous forme lyophilisée; et, dans ce cas, la trousse comprend de plus un flacon contenant un tampon de dilution du chromogène.

Ainsi la trousse comportera tous les éléments nécessaires pour établir la courbe standard d'étalonnage et le dosage des échantillons.

Le procédé de l'invention peut également être utilisé pour le dosage immunologique quantitatif de l'α-foetoprotéine. L'α-foetoprotéine est une glycoprotéine synthétisée par les cellules du foie foetal et du sac vitellin. C'est le constituant majeur du sérum foetal. On en trouve également dans le liquide amniotique, et dans le sérum maternel. Dans le liquide amniotique, le taux maximum est atteint vers la 15è semaine (20 à 50 $\mu$/ml) et dans le sérum maternel vers la 34è semaine ($>200$ ng/ml). L'utilisation du dosage de l'α-foetoprotéine est particulièrement utile dans les cas suivants.

En obstétrique l'I-fétoprotéine permet le diagnostic anténatal des malformations du tube neural par des dosages sur sang maternel, puis sur liquide amniotique réalisés vers les, 16 à 18 semaines de gestation. Par ailleurs, un taux exagéré d'α-foetoprotéine peut être indicateur de souffrance foetale, de grossesse multiple, tandis qu'un taux faible peut faire suspecter une toxémie, un retard de croissance ou une tumeur placentaire.

En cancérologie, l'α-foetoprotéine aide au diagnostic et permet le suivi post-thérapeutique des carcinomes hépatocellulaires notamment sur cirrhose, des tératocarcinomes de toutes localisations mais surtout ovariens, testicularies et sacrococcygiens, et des métastases hépatiques de différents autres cancers notamment digestifs.

En hépatologie, l'α-foetoprotéine témoigne du processus de régénération intense dans les hépatites virales.

En pédiatrie, l'α-foetoprotéine permet de différencier l'atrésie biliaire et hépatite néonatale et d'affirmer une tyrosinose héréditaire.

Le procédé, selon l'invention, de dosage de l'α-foetoprotéine présente dans un échantillon de liquide amniotique, de sérum ou de plasma, se caractérise en ce qu'il comprend les étapes suivantes:

1) mettre en contact l'échantillon contenant l'α-foetoprotéine à doser avec un composé conjugué d'un anticorps spécifique de l'α-foetoprotéine à doser et d'une enzyme, en quantité telle que l'α-foetoprotéine à doser se trouve en excès par rapport à l'anticorps;
2) mettre en contact au moins une partie du milieu réactionnel ainsi obtenu avec une phase solide sur laquelle est fixé un anticorps spécifique de l'α-foetoprotéine à doser;
3) séparer le milieu réactionnel de la phase solide; et
4) déterminer la teneur en enzyme de la phase solide.

Selon une variante de mise en oeuvre de ce procédé on réalise simultanément les étapes 1) et 2) en mettant en contact l'échantillon contenant l'α-foetoprotéine à doser avec le composé conjugué de l'anticorps spécifique de l'α-foetoprotéine et d'une enzyme et avec la phase solide sur laquelle est fixé un anticorps spécifique de l'α-foetoprotéine à doser.

Selon un mode préféré de mise en oeuvre de ce procédé, l'anticorps du composé conjugué est un anticorps monoclonal, et l'anticorps fixé sur la phase solide est une immunoglobuline anti α-foetoprotéine.

De préférence, l'enzyme utilisée est la peroxydase de Raifort.

Comme dans le cas du dosage des lipoprotéines, les éléments nécessaires pour réaliser ce dosage peuvent être disposés dans une trousse qui comprend:

- un système de tubes comportant chacun une phase solide revêtue dans les mêmes conditions d'immunoglobulines anti α-foetoproténe humaine;
- une série de flacons contenant des échantillons standard couvrant la gamme de 50 a 1000 ng/ml d'α-foetoprotéine;
- un flacon contenant le composé conjugué provenant du couplage d'un anticorps monoclonal spécifique de l'α-foetoprotéine avec une enzyme; et
- un flacon contenant un chromogène pour la révélation enzymatique.

Comme peur le dosage des lipoprotéines, les tubes peuvent comporter une phase solide constituée par un dispositif à ailettes et le chromogène peut être conditionné sous forme lyophilisée. Dans ce dernier cas, la trousse comprend de plus, un flacon contenant un tampon de dilution du chromogène.

Les exemples suivants donnés bien entendu à titre non limitatif en référence aux figures annexées illustrent la mise en oeuvre du procédé de l'invention pour le dosage d'apolipoprotéine B et pour le dosage d'α-foetoprotéine:

- la figure 1 représente schématiquement en coupe verticale un dispositif de dosage selon l'invention,

6

- les figures 2 et 3 représentent les courbes standard de dosage des lipoprotéines B obtenues selon le procédé de l'invention,
- la figure 4 représente la courbe standard de dosage des lipoprotéines B obtenue par un procédé l'art antérieur, et
- la figure 5 représente la courbe standard de dosage de l'α-foetoprotéine obtenue en utilisant des anticorps monoclonaux conformément au procédé de l'invention.

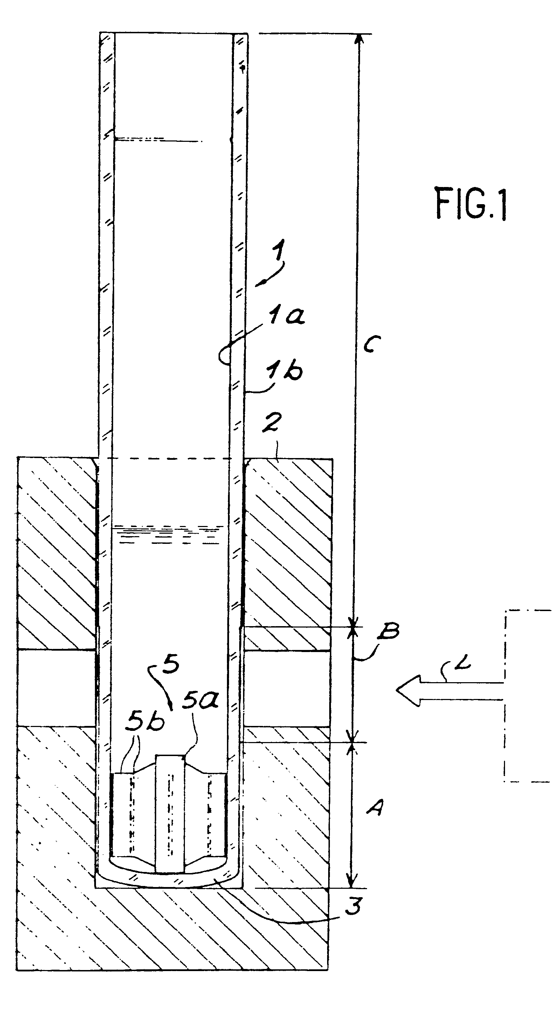

Sur la figure 1, on voit que le dispositif de dosage utilisé dans les exemples qui suivent, est constitué par un tube transparent 1 presque cylindrique de section circulaire qui est fermé à sa base par un fond plat 3. Ce tube comprend successivement depuis sa base une première zone de réaction A dans laquelle est engagée et immobilisée une pièce 5 destinée à fixer l'anticorps spécifique de la protéine à doser.

Cette pièce comprend un manchon cylindrique plein 5a dont l'extrémité inférieure repose sur le fond 3 du tube 1 et des ailettes longitudinales 5b dont les bords externes viennent en contact avec la surface interne 1a du tube 1, ce qui permet d'assurer l'immobilisation de la pièce dans le tube puisque cette pièce est réalisée en matériau élastique, par exemple, en polyamide. La hauteur de cette pièce 5 determine la hauteur de la zone de réaction A du tube 1.

Comme on peut le voir sur le dessin, le fait d'avoir un fond plat 3 permet de limiter les quantités de réactif à utiliser, qui doivent être juste suffisantes pour immerger totalement la pièce 5. Au-dessus de la première zone de réaction A, le tube comporte une seconde zone de lecture B, puis une zone C de hauteur plus importante permettant de réaliser les étapes de lavage. Dans la seconde zone de lecture B, le tube présente une très bonne qualité optique qui est obtenue en particulier grâce au fait que la surface interne 1a et la surface externe 1b de la paroi du tube 1 sont bien centrées l'une par rapport à l'autre, dans cette zone. Ainsi, dans cette zone du tube, l'épaisseur e de la paroi est homogene et constante.

Le tube 1 est réalisé en matière plastique, par exemple en polyméthacrylate de méthyle et il est obtenu de préférence par moulage par injection.

## Exemple 1. Dosage des apolipoprotéines B

Pour réaliser ce dosage, on prépare tout d'abord une gamme de 6 échantillons standard ayant des concentrations connues en lipoprotéines B allant de 0,5 mg/ml à 0,005 mg/ml par dilution au 1/2, 1/10, 1/20, 1/50, 1/100 et 1/200 d'une solution mère de lipoprotéines B à 1 g/l dans un tampon borate 0,05 M pH = 8, NaCl 150 mM, KCl 20 mM, contenant en outre 15 % (v/v) de sérum de mouton normal délipoprotéiné par ultracentrifugation.

L'anticorps spécifique des lipoprotéines B humaines est obtenu chez le mouton par immunisation d'un mouton au moyen de lipoprotéines B humaines analogues à celles utilisées pour la préparation des échantillons standard. Les immunoglobulines sont obtenues à partir du sérum du mouton immunisé et on les purifie par précipitation à l'acide caprylique, séparation par centrifugation, récupération du surnageant par filtration, puis dialyse du surnageant pendant une nuit à 4°C contre un tampon acétate 0,1 M à pH 5,7. On met ensuite la solution en contact pendant 15 min avec du gel DEAE A 50 à raison de 8 g de DEAE A 50 tamponné (dans la même solution) par gramme de protéines. On recueille ensuite par filtration une solution contenant des immunoglobulines dont la pureté peut être testée par électrophorèse.

On prépare ensuite le composé conjugué de l'anticorps spécifique des lipoprotéines B et d'une enzyme en couplant les immunoglobulines ainsi obtenues et l'enzyme constitué par la peroxydase de Raifort de Boehringer (Grade 1 R.Z. = 3) selon la technique au periodate de sodium décrite par Wilson et Nakane. On purifie ensuite le composé conjugué ainsi obtenu par filtration sur gel en utilisant comme gel l'Ultrogel Ac A 44 et en réalisant l'élution par un tampon PBS (50 mM; pH = 7,2). Cette purification permet essentiellement d'éliminer les traces d'enzyme libre qui pourraient fortement interférer dans le dosage. Le composé conjugué ainsi obtenu est ensuite dilué au 1/200 dans un tampon borate 0,05 M; pH = 8,0, NaCl 150 mM, KCl 20 mM contenant en outre 15 % (v/v) de sérum de mouton normal délipoprotéiné par ultracentrifugation.

Pour réaliser le dosage, on utilise les tubes en polyméthacrylate de méthyle représentés sur la figure 1, au fond desquels sont engagés et immobilisés des dispositifs à ailettes réalisés en polyamide sur lesquels sont fixés les anticorps spécifiques des lipoprotéines B.

Pour fixer ces anticorps sur la phase solide constituée par le dispositif à ailettes du tube, on introduit dans le tube 1 ml d'une solution d'immunoglobuline à 50 µg/ml dans un tampon phosphate (0,05 M; pH = 7,0) pendant 4 heures à la température ambiante, en utilisant les immunoglobulines obtenues chez le mouton après les avoir activées selon la methode décrite par Eiji Ishikawa, Yoshitaka Hamaguchi, Masayoshi Imagawa. Au bout des 4 heures, le tube est lavé trois fois avec le tampon phosphate, puis séché sous vide à 37°C et conservé ensuite à 4°C à l'abri de l'humidité.

On réalise ensuite le dosage de la façon suivante.

On introduit dans un premier tube en plastique sans dispositif à ailettes, 0,5 ml d'échantillon standard ou d'échantillon à doser et 0,5 ml du composé conjugué prédilué au 1/200 dans le tampon borate et on laisse incuber à la température ambiante. Après incubation pendant 1 h 30 min, on verse le contenu de ce premier tube ou tout au moins une partie de ce contenu (au moins 0,450 ml) dans l'un des tubes en plastique dont le

7

dispositif à ailettes est revêtu de l'anticorps spécifique des lipoprotéines B, et on maintient le milieu liquide au contact des ailettes pendant deux heures, à la temperature ambiante. On aspire alors la phase liquide du tube et on lave le tube tout d'abord à l'eau permutée contenant 0,1 % de Tween 20, puis deux fois à l'eau permutée.

Ces lavages se font en remplissant le tube de 2 ml de liquide et en le vidant par aspiration.

Après ces lavages, on verse dans le tube 0,5 ml de la solution de chromogène reconstituée extemporanément par addition de 16 ml d'un tampon substrat contenant 0,92 % (p/v) d'acide citrique monohydraté, 0,01 % (p/v) de thimérosal et 0,02 % (v/v) de $H_2O_2$ à 30 % (Merck) à un mélange obtenu par lyophilisation d'une solution à 10 mg/ml d'orthophénylène diamine, 2HCl (Sigma) dans 3,2 ml de phosphate ($Na_2HPO_4$, $2H_2O$ à 100 mg/ml). On laisse se développer la réaction enzymatique pendant une demi-heure à la température ambiante, et on arrête le processus par addition de 2 ml de solution d'acide oxalique 1 N. On détermine alors la densité optique de la solution par spectrophotométrie à 492 nm, en introduisant le tube 1 dans le spectrophotomètre 2 de telle sorte que le faisceau lumineux L traverse le tube 1 au niveau de la zone de lecture B du tube 1.

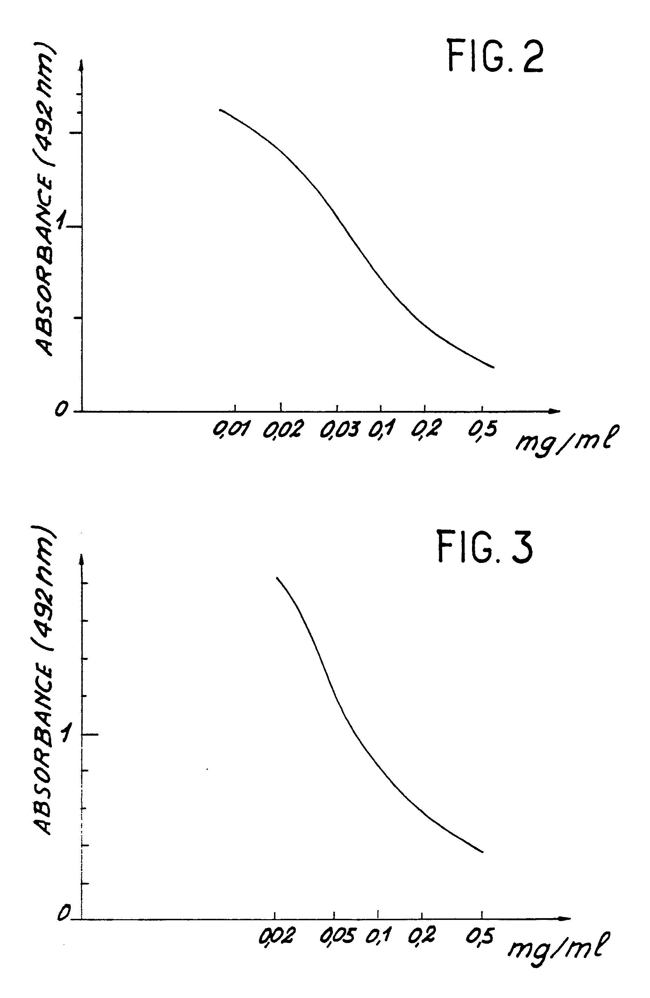

On répète les opérations de dosage pour tous les échantillons standard et les résultats obtenus sont donnés dans le tableau 1 qui suit et sur la figure 2 qui représente la courbe standard donnant les densités optiques en fonction de la teneur en lipoprotéines B des échantillons.

On réalise également les mêmes opérations de dosage sur l'échantillon à doser et on repère alors sur la courbe standard à quelle concentration en lipoprotéines B correspond la densité optique mesurée sur cet échantillons.

**Tableau 1**

| Dilution | Concentration (en mg/ml) | D.O. |
|---|---|---|
| 1/2 | 0,5 | 0,28 |
| 1/5 | 0,2 | 0,49 |
| 1/10 | 0,1 | 0,74 |
| 1/20 | 0,05 | 1,01 |
| 1/50 | 0,02 | 1,42 |
| 1/100 | 0,01 | 1,60 |

**Exemple 2. Dosage des apolipoprotéines**

Pour réaliser ces dosages, on prépare comme dans l'exemple 1, une gamme de six échantillons standard ayant des concentrations connues en lipoprotéines B allant de 0,5 à 0,01 mg/ml par dilution au 1/2, 1/5, 1/10, 1/20, 1/50 et 1/100 d'une solution-mère de lipoprotéines B à 1 g/l dans un tampon-borate 0,05 M, pH = 8 NaCl 150 mM, KCl 20 mM, contenant en outre 15 % (v/v) de sérum de mouton normal délipoprotéiné par ultracentrifugation.

Pour ce dosage, on utilise les mêmes tubes, le même anticorps et le même composé conjugué que dans l'exemple 1, l'anticorps étant fixé sur la phase solide constituée par le dispositif à ailettes du tube. On réalise ensuite le dosage de la façon suivante:

On introduit dans le tube comportant le dispositif à ailettes sur lequel est fixé l'anticorps, 100 μl d'échantillon standard et 1 ml du composé conjugué prédilué au 1/2000 et on laisse incuber pendant 2 h à 37°C. On aspire alors la phase liquide du tube et on lave le tube comme dans l'exemple 1. On effectue également une opération de révélation enzymatique de la phase solide dans les mêmes conditions que celles de l'exemple 1 en utilisant les mêmes produits.

On répète les opérations de dosage pour tous les échantillons standard. Les résultats obtenus sont donnés dans le tableau 2 qui suit et sur la figure 3 qui représente la courbe standard donnant les densités optiques en fonction de la teneur en lipoprotéines B des échantillons. On réalise également les mêmes opérations de dosage sur l'échantillon à doser mais en utilisant 10 μl d'échantillon et 1 ml du composé conjugué prédilué au 1/2000.

**Tableu 2**

| Dilution | Concentration (en mg/ml) | D.O. |
|---|---|---|
| 1/2 | 0,5 | 0,35 |
| 1/5 | 0,2 | 0,58 |
| 1/10 | 0,1 | 0,84 |
| 1/20 | 0,05 | 1,24 |
| 1/50 | 0,02 | 1,850 |
| 1/100 | 0,01 | 2,000 |

EP 0 157 672 B1

**Exemple 3. Dosage des lipoprotéines B**

A titre comparatif, on donne, ci-après, les résultats obtenus avec une méthode de dosage immunoenzymatique classique utilisant les mêmes réactifs mais comprenant les étapes suivantes:

1° Incubation de l'échantillon standard ou de l'échantillon à doser avec la phase solide sur laquelle est fixé l'anticorps
Dans des tubes comportant le dispositif à ailettes revêtu des anticorps spécifiques des lipoprotéines B, on met à incuber 0,5 ml de standard prédilué au 1/100, 1/500, 1/1000, 1/2000, 1/4000, 1/5000, 1/10000, 1/20000 pendant une heure à la température ambiante. On aspire alors le contenu du tube et on le lave une première fois avec une solution de Tween 20® à 0,1 %, puis une deuxième et une troisième fois avec de l'eau permutée.

2° Incubation du composé conjugué
On ajoute alors dans le tube 0,5 ml du composé conjugué anticorps antilipoprotéines humaines-peroxydase de Raifort dilué au 1/1500 et on laisse incuber pendant deux heures à la température ambiante. On aspire ensuite la phase liquide et on lave le tube comme précédemment, une première fois avec une solution de Tween 20® à 0,1 % et deux fois avec de l'eau permutée.

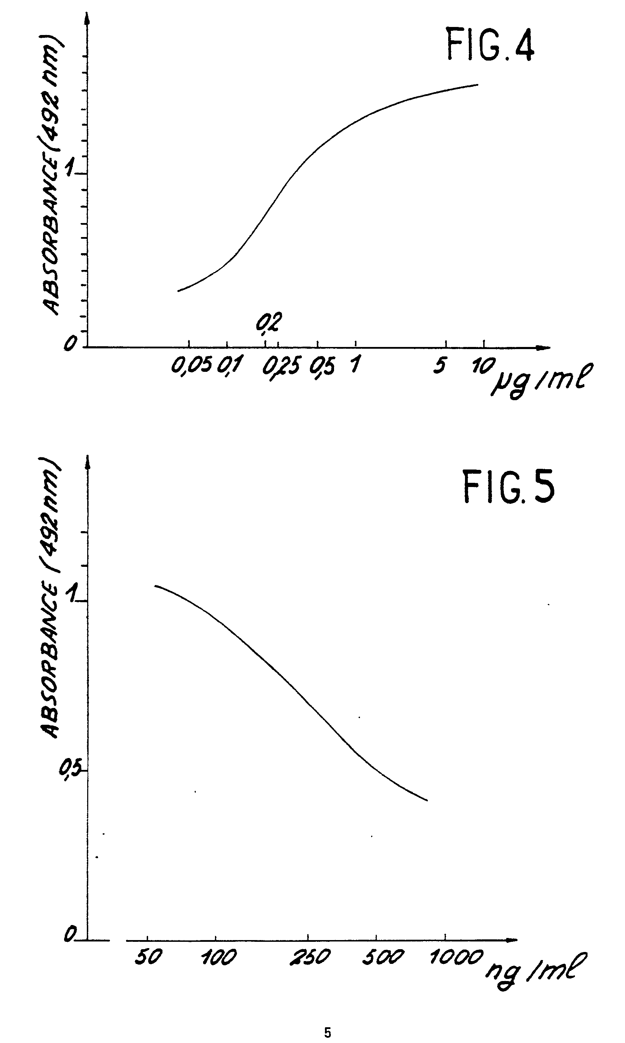

3° Dosage de l'enzyme fixée sur la phase solide
La révélation enzymatique se fait par addition du même chromogène et dans les mêmes conditions. On réalise également la lecture à 492 nm. Les résultats obtenus sont donnés dans le tableau 3 qui suit et sur la figure 4 qui représente la courbe standard obtenue dans ces conditions.
Avec cette méthode classique, un échantillon à doser doit être dilué au 1/5000 ou 1/10000 alors que, dans le procédé de l'invention, l'échantillon peut ne pas subir de dilution préalable si la prise d'essai est de 20 µl ou être seulement dilué au 1/25 ou au 1/50 pour des prises d'essai de 1 ml.

**Tableau 3**

| Dilution | Concentration (en µg/ml) | D.O. |
|----------|--------------------------|------|
| 1/100 | 10 µg/ml | 1,70 |
| 1/500 | 5 | 1,65 |
| 1/1000 | 1 | 1,50 |
| 1/2000 | 0,5 | 1,32 |
| 1/4000 | 0,25 | 1,05 |
| 1/5000 | 0,2 | 0,85 |
| l/10000 | 0,1 | 0,51 |
| 1/20000 | 0,050 | 0,37 |
| | blanc | 0,16 |

**Exemple 4. Dosage de l'$\alpha$-foetoprotéine**

Cet exemple illustre le dosage d'alpha-foetoprotéine par le procédé de l'invention en utilisant comme premier anticorps un anticorps monoclonal spécifique de l'$\alpha$-foetoprotéine et comme deuxième anticorps fixé sur la phase solide des immunoglobulines anti $\alpha$-foetoprotéine humaine obtenues chez la chevre.
On prépare tout d'abord une gamme de 4 échantillons standard ayant des concentrations connues en $\alpha$-foetoprotéines allant de 50 ng/ml à 1000 ng/ml obtenus par dilution de liquides amniotiques dans un tampon phosphate (50 mM; pH = 7,4) et contenant en outre 5 % (v/v) de sérum de mouton normal et 0,01 % (v/v) de thimérosal.
On utilise un anticorps monoclonal $K6B_1$ obtenu par fusion de splénocytes de souris BALB/C immunisées avec de l'$\alpha$-foetoprotéine d'origine humaine et de cellules de myélome selon une technique dérivée de celle de Kohler G. et Milstein C., décrite par Sertour J., Bellet D., Salard J.L. et Marchand J. "Polyclonal or Monoclonal Antibodies in an AFP Assay. Protides of the Biological Fluids. 31st Colloquium", Bruxelles 1983 - Ed. Dr H. Peeters-Pergamon Press, Oxford and New York.
On prépare ensuite le composé conjugué de l'anticorps monoclonal spécifique de l'$\alpha$-foetoprotéine et d'une enzyme en couplant l'anticorps monoclonal avec la peroxydase de Raifort selon la technique utilisée dans l'exemple 1. On purifie également le composé conjugué dans les mêmes conditions que celles de l'exemple 1, puis on dilue celui-ci au 1/20000 dans un tampon phosphate (50 mM; pH = 7,4), contenant en outre 5 % (v/v) de sérum de mouton normal et 0,01 % de thimérosal.
Pour réaliser le dosage, on utilise les tubes en polystyrène représentés sur la figure 1, au fond desquels est engagé et immobilisé un dispositif à ailettes réalisé en polyamide sur lequel est fixé le deuxième anticorps spécifique de l'$\alpha$-foetoprotéine.

9

Pour fixer cet anticorps sur la phase solide constituée par le dispositif à ailettes du tube, on introduit dans le tube 1 ml d'une solution d'immunoglobulines à 50 μg/ml dans un tampon phosphate (0,05 M; pH = 7,0) pendant 4h à la température ambiante, en utilisant des immunoglobulines anti-alphafoetoprotéine obtenues chez la chèvre après les avoir activées selon la méthode décrite par Eiji Ishikawa, Yoshitaka Hamaguchi, Masayoshi Imagawa.

Au bout des 4h, le tube est lavé 3 fois avec le tampon phosphate puis séché sous vide à 37°C et conservé ensuite à 4°C à l'abri de l'humidité.

On réalise ensuite le dosage de la façon suivante:

On introduit dans un premier tube en plastique sans dispositif à ailettes 0,5 ml d'échantillon standard ou d'échantillon à doser et 0,5 ml du composé conjugué prédilué au 1/20000 dans le tampon phosphate, et on laisse incuber à 37°C. Après incubation pendant 1 h 30 min, on verse le contenu de ce premier tube ou tout au moins une partie de ce contenu (au moins 0,450 ml) dans l'un des tubes en plastique dont le dispositif à ailettes est revêtu de l'anticorps spécifique constitué par les immunoglobulines anti-alphafoetoprotéine obtenues chez la chèvre, et on maintient le milieu liquide au contact des ailettes pendant 1 h à 37°C. On aspire alors la phase liquide du tube et on lave le tube tout d'abord à l'eau permutée contenant 0,1 % de Tween 20®, puis deux fois à l'eau permutée.

Après ces lavages, on verse dans le tube 0,5 ml de la solution de chromogènes utilisée dans l'exemple 1 et on effectue la révélation enzymatique dans les mêmes conditions que celles de l'exemple 1.

Les résultats obtenus sont donnés sur la figure 5 qui représente la courbe standard obtenue dans ces conditions.

## Revendications

1. Procédé de dosage immunologique quantitatif d'un substance, comprenant les étapes suivantes:
   1) mettre en contact un échantillon contenant la substance à doser avec un réactif immunoactif marqué spécifique de la substance à doser;
   2) mettre en contact au moins une partie du milieu réactionnel ainsi obtenu avec une phase solide sur laquelle est fixé un réactif immunoactif spécifique de la substance à doser;
   3) séparer le milieu réactionnel de la phase solide et;
   4) déterminer la teneur en réactif immunoactif marqué de la phase solide, caractérisé en ce que dans la première étape, on utilise une quantité de réactif immunoactif telle que la substance à doser se trouve en excès par rapport au réactif immunoactif marqué.

2. Procédé selon la revendication 1, caractérisé en ce que l'on réalise simultanément les étapes 1) et 2) en mettant en contact la substance à doser simultanément avec le réactif immunoactif marqué et avec la phase solide sur laquelle est fixé le réactif immunoactif.

3. Procédé selon l'une quelconque des revendications 1 et 2, caractérisé en ce que la substance à doser est un antigène ou un haptène.

4. Procédé selon la revendication 3, caractérisé en ce que le réactif immunoactif marqué est un anticorps marqué par un radioélément.

5. Procédé selon la revendication 3, caractérisé en ce que le réactif immunoactif marqué est un composé conjugué d'une enzyme et d'un anticorps spécifique de l'antigène ou de l'haptène à doser.

6. Procédé selon la revendication 3, caractérisé en ce que le réactif immunoactif marqué est un anticorps marqué par un élément fluorescent.

7. Procédé selon l'une quelconque des revendications 3 à 6, caractérisé en ce que l'un au moins des réactifs immunoactifs est un anticorps monoclonal.

8. Procédé selon l'une quelconque des revendications 1 et 2, caractérisé en ce que la substance à doser est une protéine plasmatique choisie dans le groupe comprenant les lipoprotéines, les immunoglobulines, les facteurs du complément et les glycoprotéines.

9. Procédé selon l'une quelconque des revendications 1 et 2, caractérisé en ce que la substance à doser est un anticorps.

10. Procédé de dosage quantitatif de lipoprotéines dans le plasma sanguin, caractérisé en ce qu'il comprend les étapes suivantes:
    1) mettre en contact un échantillon de plasma sanguin contenant les lipoprotéines à doser avec un composé conjugué d'un anticorps spécifique de ces lipoprotéines et d'une enzyme, en quantité telle que les lipoprotéines à doser se trouvent en excès par rapport à l'anticorps;
    2) mettre en contact au moins une partie du milieu réactionnel ainsi obtenu avec une phase solide sur laquelle est fixé le même anticorps spécifique des lipoprotéines à doser;
    3) séparer le milieu réactionnel de la phase solide, et
    4) déterminer la teneur en enzyme de la phase solide.

11. Procédé selon la revendication 10, caractérisé en ce que l'on réalise simultanément les étapes 1) et 2) en mettant en contact l'échantillon de plasma sanguin contenant les lipoprotéines à doser simultanément avec le composé conjugué d'un anticorps spécifique de ces lipoprotéines et d'une enzyme et avec la phase solide sur laquelle est fixé le même anticorps spécifique des lipoprotéines à doser.

EP 0 157 672 B1

12. Procédé selon l'une quelconque des revendications 10 et 11, caractérisé en ce que l'enzyme est la peroxydase de Raifort.

13. Trousse pour le dosage de lipoprotéines par mise en oeuvre du procédé selon l'une quelconque des revendications 10 à 12, caractérisée en ce qu'elle comprend:
- un système de tubes comportant chacun une phase solide revêtue dans les mêmes conditions d'immunoglobulines antilipoprotéines humaines;
- une série de flacons contenant des échantillons standard couvrant la gamme de 0,1 à 0,005 g/l de lipoprotéines;
- 1 flacon contenant le composé conjugué provenant du couplage des immunoglobulines antilipoprotéines humaines avec une enzyme; et
- un flacon contenant un chromogène pour la révélation enzymatique.

14. Procédé de dosage quantitatif de l'α-foetoprotéine présente dans un échantillon de sérum, de plasma ou de liquide amniotique, caractérisé en ce qu'il comprend les étapes suivantes:
1) mettre en contact l'échantillon contenant l'α-foetoprotéine à doser avec un composé conjugué d'un anticorps spécifique de l'α-foetoprotéine à doser et d'une enzyme, en quantité telle que l'α-foetoprotéine à doser se trouve en excès par rapport à l'anticorps;
2) mettre en contact au moins une partie du milieu réactionnel ainsi obtenu avec une phase solide sur laquelle est fixé un anticorps spécifique de l'α-foetoprotéine à doser;
3) séparer le milieu réactionnel de la phase solide; et
4) déterminer la teneur en enzyme de la phase solide.

15. Procédé selon la revendication 14, caractérisé en ce que l'on réalise simultanément les étapes 1) et 2) en mettant en contact l'échantillon contenant l'α-foetoprotéine à doser simultanément avec le composé conjugué d'un anticorps spécifique de l'α-foetoprotéine à doser et d'une enzyme et avec la phase solide sur laquelle est fixé un anticorps spécifique de l'α-foetoprotéine à doser.

16. Procédé selon l'une quelconque des revendications 14 et 15, caractérisé en ce que l'anticorps du composé conjugué est un anticorps monoclonal.

17. Procédé selon l'une quelconque des revendications 14 à 16, caractérisé en ce que l'anticorps fixé sur la phase solide est une immunoglobuline anti α-foetoprotéine.

18. Procédé selon l'une quelconque des revendications 14 à 17, caractérisé en ce que l'enzyme est la peroxydase de Raifort.

19. Trousse pour le dosage de l'α-foetoprotéine par mise en oeuvre du procédé selon l'une quelconque des revendications 14 à 18, caractérisée en ce qu'elle comprend:
- un système de tubes comportant chacun une phase solide revêtue dans les mêmes conditions d'immunoglobulines anti α-foetoprotéine humaine;
- une série de flacons contenant des échantillons standard couvrant la gamme de 50 à 1000 ng/ml d'α-foetoprotéine;
- un flacon contenant le composé conjugué provenant du couplage d'un anticorps monoclonal spécifique de l'α-foetoprotéine avec une enzyme et;
- un flacon contenant un chromogène pour la révélation enzymatique.

20. Trousse selon l'une quelconque des revendication 13 et 19, caractérisée en ce qu'elle comprend de plus un flacon contenant un tampon de dilution du chromogène.

## Claims

1. Process for quantitatively immunoassaying a substance, comprising the following stages:
1) contacting a sample containing the substance to be assayed with a labelled immunoactive reagent specific to said substance, the immunoactive reagent quantity being such that the substance to be assayed is in excess compared with that labelled immunoactive reagent;
2) contacting at least part of the thus obtained reaction medium with a solid phase, to which is fixed an immunoactive reagent specific to the substance to be assayed;
3) separating the reaction medium from the solid phase; and
4) determining the labelled immunoactive reagent content of the solid phase, characterized in that in the first stage use is made of an immunoactive reagent quantity such that the substance to be assayed is in excess compared with the labelled immunoactive reagent.

2. Process according to claim 1, characterized in that stages 1) and 2) are simultaneously performed by contacting the substance to be assayed simultaneously with the labelled immunoactive reagent and with the solid phase to which is fixed said reagent.

3. Process according to either of the claims 1 and 2, characterized in that the substance to be assayed is an antigen or a hapten.

4. Process according to claim 3, characterized in that the labelled immunoactive reagent is an antibody labelled by a radioelement.

5. Process according to claim 3, characterized in that the labelled immunoactive reagent is a conjugate compound of an enzyme and an antibody specific to the antigen or hapten to be assayed.

11

6. Process according to claim 3, characterized in that the labelled immunoactive reagent is an antibody labelled by a fluorescent element.

7. Process according to any one of the claims 3 to 6, characterized in that at least one of the immunoactive reagents is a monoclonal antibody.

8. Process according to either of the claims 1 and 2, characterized in that the substance to be assayed is a plasma protein chosen from the group including lipoprotein, immunoglobulins, complement factors and glycoproteins.

9. Process according to either of the claims 1 and 2, characterized in that the substance to be assayed is an antibody.

10. Process for quantitatively assaying lipoproteins in the blood plasma, characterized in that it comprises the following stages:

1) contacting a blood plasma sample containing the lipoproteins to be assayed with a conjugate compound of an antibody specific to said lipoproteins and an enzyme in quantities such that the lipoproteins to be assayed are in excess compared with the antibody;

2) contacting at least part of the thus obtained reaction medium with a solid phase to which is fixed the same antibody specific to the lipoproteins to be assayed;

3) separating the reaction medium from the solid phase; and

4) determining the enzyme content of the solid phase.

11. Process according to claim 10, characterized in that stages 1) and 2) are simultaneously performed by contacting the blood plasma sample containing the lipoproteins to be assayed simultaneously with the conjugate compound of an antibody specific to these lipoproteins and an enzyme, as well as with the solid phase to which is fixed the same antibody specific to the lipoproteins to be assayed.

12. Process according to either of the claims 10 and 11, characterized in that the enzyme is the Raifort peroxidase.

13. Kit for assaying lipoproteins by performing the process according to any one of the claims 10 to 12, characterized in that it comprises:

- a system of tubes each having a solid phase coated under the same conditions with human antilipoprotein immunoglobulins;

- a series of bottles containing standard lipoproteins samples covering the range 0.1 to 0.005 g/l;

- one bottle containing the conjugate compound resulting from the coupling of human antilipoproteins immunoglobulins with an enzyme; and

- a bottle containing a chromogen for enzymatic detection.

14. Process for quantitatively assaying the α-foetoprotein present in a sample of serum, plasma or amniotic fluid, characterized in that it comprises the following stages:

1) contacting the sample containing the α-foetoprotein to be assayed with a conjugate compound of an antibody specific to the α-foetoprotein to be assayed and an enzyme, in a quantity such that the α-foetoprotein to be assayed is in excess compared with the antibody;

2) contacting at least part of the thus obtained reaction medium with a solid phase to which is fixed an antibody specific to the α-foetoprotein to be assayed;

3) separating the reaction medium from the solid phase; and

4) determining the enzyme content of the solid phase.

15. Process according to claim 14, characterized in that stages 1) and 2) are simultaneously performed by contacting the sample containing the α-foetoprotein to be assayed simultaneously with the conjugate compound of an antibody specific to the α-foetoprotein to be assayed and an enzyme, as well as with the solid phase to which is fixed an antibody specific to the α-foetoprotein to be assayed.

16. Process according to either of the claims 14 and 15, characterized in that the antibody of the conjugate compound is a monoclonal antibody.

17. Process according to any one of the claims 14 to 16, characterized in that the antibody fixed to the solid phase is an anti-α-foetoprotein immunoglobulin.

18. Process according to any one of the claims 14 to 17, characterized in that the enzyme is the Raifort peroxidase.

19. Kit for assaying α-foetoprotein by performing the process according to any one of the claims 14 to 18, characterized in that it comprises:

- a system of tubes each having a solid phase coated under the same conditions with human anti α-foetoprotein immunoglobulins;

- a series of bottles containing standard samples having the α-foetoprotein range of 50 to 1000 ng/ml;

- a bottle containing the conjugate compound resulting from the coupling of a monoclonal antibody specific to the α-foetoprotein with an enzyme; and

- a bottle containing a chromogen for enzymatic detection.

20. Kit according to either of the claims 13 and 19, characterized in that it also comprises a bottle containing a chromogen dilution buffer.

12

**EP 0 157 672 B1**

**Patentansprüche**

1. Verfahren zur immunologischen quantitativen Bestimmung einer Substanz, umfassend die folgenden Schritte:

1) eine Probe, die die zu messende Substanz enthält, wird mit einem immunaktiven Reagenz in Berührung gebracht, das mit der zu messenden Substanz speziell gekennzeichnet ist;

2) wenigstens ein Teil des so erhaltenen Reaktionsmittels wird mit einer festen Phase in Berührung gebracht, auf der ein immunaktives Reagenz befestigt ist, das für die zu messende Substanz spezifisch ist;

3) das Reaktionsmittel wird von der festen Phase getrennt; und

4) der Gehalt an mit der festen Phase markiertem immunaktivem Reagenz wird ermittelt, dadurch gekennzeichnet daß man im ersten Schritt eine solche Menge an immunaktivem Reagenz verwendet, daß die zu messende Substanz sich im Überschuß gegenüber dem markierten immunaktiven Reagenz befindet.

2. Verfahren nach Anspruch 1, dadurch gekennzeichnet, daß man gleichzeitig die Schritte 1) und 2) ausführt, indem man die zu messende Substanz mit dem markierten immunaktiven Reagenz und mit der festen Phase, auf der das immunaktive Reagenz befestigt ist, in Berührung bringt.

3. Verfahren nach einem der Ansprüche 1 und 2, dadurch gekennzeichnet, daß die zu messende Substanz ein Antigen oder ein Hapten ist.

4. Verfahren nach Anspruch 3, dadurch gekennzeichnet, daß das markierte immunaktive Reagenz ein Antikörper ist, der durch ein Radioelement markiert ist.

5. Verfahren nach Anspruch 3, dadurch gekennzeichnet, daß das markierte immunaktive Reagenz eine gekoppelte Verbindung eines Enzyms und eines Antikörpers ist, der für das zu messende Antigen oder Hapten spezifisch ist.

6. Verfahren nach Anspruch 3, dadurch gekennzeichnet, daß das markierte immunaktive Reagenz ein Antikörper ist, der durch ein fluoreszierendes Element markiert ist.

7. Verfahren nach einem der Ansprüche 3 bis 6, dadurch gekennzeichnet, daß wenigstens eines der immunaktiven Reagenzien ein monoklonaler Antikörper ist.

8. Verfahren nach einem der Ansprüche 1 und 2, dadurch gekennzeichnet, daß die zu messende Substanz ein Proteinplasma ist, das aus der Gruppe ausgewählt ist, die die Lipoproteine, die Immunglobuline, die Komplementfaktoren und die Glycoproteine enthält.

9. Verfahren nach einem der Ansprüche 1 und 2, dadurch gekennzeichnet, daß die zu messende Substanz ein Antikörper ist.

10. Verfahren zur quantitativen Bestimmung von Lipoproteinen im Blutplasma, dadurch gekennzeichnet, daß es die folgenden Schritte enthält:

1) eine Blutplasmaprobe, die die zu messenden Lipoproteine enthält, wird mit einer gekoppelten Zusammensetzung in Berührung gebracht, die aus einem für diese Lipoproteine spezifischen Antikörper und aus einem Enzym besteht, in einer solchen Menge, daß die zu messenden Lipoproteine sich gegenüber den Antikörpern im überschuß befinden;

2) wenigstens ein Teil des so erhaltenen Reaktionsmittels wird mit einer festen Phase in Berührung gebracht, auf der derselbe für die zu messenden Lipoproteine spezifische Antikörper befestigt ist,

3) das Reaktionsmittel wird von der festen Phase abgetrennt, und

4) der Gehalt an Enzym der festen Phase wird ermittelt.

11. Verfahren nach Anspruch 10, dadurch gekennzeichnet, daß man die Schritte 1) und 2) gleichzeitig ausführt, indem man die Blutplasmaprobe, die die zu messenden Lipoproteine enthält, gleichzeitig mit der gekoppelten Zusammensetzung, die aus einem für diese Lipoproteine spezifischen Antikörper und aus einem Enzym besteht, und mit der festen Phase, auf der derselbe für die zu messenden Lipoproteine spezifische Antikörper befestigt ist, in Berührung bringt.

12. Verfahren nach einem der Ansprüche 10 und 11, dadurch gekennzeichnet, daß das Enzym Raifort-Peroxydase ist.

13. Garnitur für die Messung von Lipoproteinen durch Ausführung des Verfahrens nach einem der Ansprüche 10 bis 12, dadurch gekennzeichnet, daß sie enthält:

- ein System von Röhren, die jeweils eine feste Phase enthalten, die unter den gleichen Bedingungen mit menschlichen Immunglobulin-Antilipoproteinen bedeckt sind;

- eine Serie von Fläschchen, die Standardproben enthalten, die einen Bereich von 0,1 bis 0,005 g/l an Lipoproteinen abdecken;

- ein Fläschchen, das die konjugierte Zusammensetzung enthält, die aus einer Verkopplung der menschlichen Immunglobulin-Antilipoproteine mit einem Enzym stammt; und

- ein Fläschchen, das ein Chromogen für die enzymatische Entwicklung enthält.

14. Verfahren zur quantitativen Ermittlung von α-Foetoprotein, das in einer Probe eines Serums, eines Plasmas oder im Fruchtwasser enthalten ist, dadurch gekennzeichnet, daß es die folgenden Schritte enthält:

1) die Probe, die das zu messende α-Foetoprotein enthält, wird mit einer verkoppelten Zusammensetzung in Berührung gebracht, die aus einem für das zu messende α-Foetoprotein spezifischen Antikörper und einem Enzym besteht, in einer solchen Menge, daß das zu messende α-Foetoprotein sich gegenüber dem Antikörper im überschuß befindet;

2) ein Teil des so erhaltenen Reaktionsmittels wird mit einer festen Phase in Berührung gebracht, auf der ein für das zu messende α-Foetoprotein spezifischer Antikörper befestigt ist;

13

3) das Reaktionsmittel wird von der festen Phase getrennt, und

4) der Gehalt an Enzym der festen Phase wird bestimmt.

15. Verfahren nach Anspruch 14, <u>dadurch gekennzeichnet</u>, daß man die Schritte 1) und 2) gleichzeitig ausführt, indem man die das zu messende α-Foetoprotein enthaltende Probe gleichzeitig mit der verkoppelten Zusammensetzung aus einem für das zu messende α-Foetoprotein spezifischen Antikörper und einem Enzym und mit der festen Phase in Berührung bringt, auf der ein für das zu messende α-Foetoprotein spezifischer Antikörper befestigt ist.

16. Verfahren nach einem der Ansprüche 14 und 15, <u>dadurch gekennzeichnet</u>, daß der Antikörper der verkoppelten Zusammensetzung ein monoklonaler Antikörper ist.

17. Verfahren nach einem der Ansprüche 14 bis 16, <u>dadurch gekennzeichnet</u>, daß der auf der festen Phase befestigte Antikörper ein Immunglobulin-Anti-α-Foetoprotein ist.

18. Verfahren nach einem der Ansprüche 14 bis 17, <u>dadurch gekennzeichnet</u>, daß das Enzym Raifort-Peroxydase ist.

19. Garnitur für die Messung von α-Foetoprotein durch Ausführung des Verfahrens nach einem der Ansprüche 14 bis 18, <u>dadurch gekennzeichnet</u>, daß sie enthält:

- ein System von Röhren, die jeweils eine feste Phase enthalten, die unter den gleichen Bedingungen von menschlichen Anti-α-Foetoprotein-Immunglobulinen bedeckt sind;

- eine Serie Fläschchen, die Standardproben enthalten, die den Bereich von 50 bis 1000 ng/ml α-Foetoprotein enthalten;

- ein Fläschchen, das die verkoppelte Zusammensetzung enthält, die aus der Kopplung eines für das α-Foetoprotein spezifischen monoklonalen Antikörpers mit einem Enzym stammt; und

- ein Fläschchen, das ein Chromogen für die enzymatische Entwicklung enthält.

20. Garnitur nach einem der Ansprüche 13 bis 19, <u>dadurch gekennzeichnet</u>, daß sie darüber hinaus ein Fläschchen enthält, das einen Tampon Verdünnung für das Chromogen enthält.

FIG.1

FIG.2

FIG.3

FIG.4

ABSORBANCE (492 nm)

1

0

0,2

0,05 0,1  0,25 0,5  1      5   10  μg/mℓ

FIG.5

ABSORBANCE (492 nm)

1

0,5

0

50      100      250      500      1000   ng/mℓ